(19) Europäisches Patentamt
European Patent Office
Office européen des brevets

(11) EP 4 509 044 A1

(12) **EUROPÄISCHE PATENTANMELDUNG**

(43) Veröffentlichungstag:
**19.02.2025 Patentblatt 2025/08**

(21) Anmeldenummer: 24195045.0

(22) Anmeldetag: **16.08.2024**

(51) Internationale Patentklassifikation (IPC):
**A61B 5/08** *(2006.01)* **A61B 5/00** *(2006.01)*
**A61B 5/397** *(2021.01)*

(52) Gemeinsame Patentklassifikation (CPC):
**A61B 5/397; A61B 5/7214;** A61B 5/0803;
A61B 5/318; A61B 5/4836; A61B 5/6823;
A61B 2562/046

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(30) Priorität: **17.08.2023 DE 102023122079**

(71) Anmelder: **Fraunhofer-Gesellschaft zur Förderung**
**der angewandten Forschung e.V.**
**80686 München (DE)**

(72) Erfinder:
• **GRAßHOFF, Jan**
**23562 Lübeck (DE)**
• **KUSCHE, Roman**
**21075 Hamburg (DE)**
• **ROSTALSKI, Philipp**
**23562 Lübeck (DE)**

(74) Vertreter: **Kuttenkeuler, David**
**SKM-IP PartGmbB**
**Oberanger 45**
**80331 München (DE)**

(54) **VERFAHREN ZUR UNTERDRÜCKUNG VON STÖRSIGNALEN IN EINER ELEKTROMYOGRAFISCHEN MESSUNG**

(57)     Die Offenbarung betrifft ein Verfahren zur Separation von Nutz- und Störsignalen in einer elektromyografischen, EMG, -Messung in einer elektrisch aktiven Region von Muskeln und/oder zur Unterdrückung von Störsignalen in der EMG-Messung, das folgende Schritte umfasst: Erfassen eines ersten Signals mit wenigstens einer ersten Sensoreinrichtung auf einer ersten Muskelgruppe, wobei das erste Signal eine erste EMG-Signalkomponente und eine erste EMG-Störkomponente aufweist; Erfassen eines zweiten Signals mit wenigstens einer zweiten Sensoreinrichtung auf der ersten Muskelgruppe, wobei das zweite Signal eine zweite EMG-Signalkomponente und eine zweite EMG-Störkomponente aufweist; Ermitteln eines kombinierten Signals auf Basis des ersten Signals und des zweiten Signals zur Unterdrückung der ersten und/oder der zweiten EMG-Störkomponente. Ferner betrifft die Offenbarung die Verwendung des Verfahrens zum Monitoring von wenigstens einer Muskelgruppe, insbesondere Atemmuskeln, vorzugsweise zum Zwerchfell-Monitoring, und/oder zur Ansteuerung eines Beatmungsgerätes, und/oder zur Ansteuerung und/oder zum Monitoring von Prothesen und/oder Orthesen, und/oder zur Bewegungsanalyse. Zudem wird ein computerimplementiertes Verfahren, ein Computerprogrammprodukt, ein computerlesbares Speichermedium und eine Vorrichtung zur Separation von Nutz- und Störsignalen in einer EMG-Messung in einer elektrisch aktiven Region von Muskeln und/oder zur Unterdrückung von Störsignalen in der EMG-Messung bereitgestellt.

Fig. 1

**Beschreibung**

TECHNISCHES GEBIET DER ERFINDUNG

Die vorliegende Erfindung betrifft ein Verfahren zur Separation von Nutz- und Störsignalen in einer elektromyografischen, EMG, -Messung in einer elektrisch aktiven Region von Muskeln und/oder zur Unterdrückung von Störsignalen in der EMG-Messung. Die vorliegende Erfindung betrifft auch die Verwendung des erfindungsgemäßen Verfahrens zum Monitoring von wenigstens einer Muskelgruppe, insbesondere Atemmuskeln, vorzugsweise zum Zwerchfell-Monitoring, und/oder zur Ansteuerung eines Beatmungsgerätes, und/oder zur Ansteuerung und/oder zum Monitoring von Prothesen und/oder Orthesen, und/oder zur Bewegungsanalyse. Zudem wird ein computerimplementiertes Verfahren, ein Computerprogrammprodukt, ein computerlesbares Speichermedium und eine Vorrichtung zur Separation von Nutz- und Störsignalen in einer EMG-Messung in einer elektrisch aktiven Region von Muskeln und/oder zur Unterdrückung von Störsignalen in der EMG-Messung bereitgestellt.

BESCHREIBUNG

Die Elektromyographie (EMG) ist ein Verfahren zur Erfassung der elektrischen Aktivität, wie sie bei der Muskelkontraktion entsteht, beispielsweise Muskelanspannungen. Wird ein Muskel erregt, so entsteht in den Muskelfasern ein Aktionspotential, welches sich aufgrund der anisotropen Leitfähigkeitseigenschaften einer Muskelfaser bevorzugt in Faserrichtung ausbreitet. Mit der EMG wird das Summenpotential aller aktiven Muskelfasern erfasst, wobei die EMG die Muskelaktionspotentiale mittels differenzieller Spannungsmessung misst. Diese Signale können entweder invasiv mittels Nadelelektroden oder nichtinvasiv mittels Oberflächenelektroden abgeleitet werden. Die in den Signalen enthaltenen Informationen werden häufig zur Ansteuerung von Prothesen oder Myoprothesen genutzt, ermöglichen jedoch auch Rückschlüsse auf den Gesundheitszustand eines Muskels oder eine Muskelgruppe.

EMG Signale, insbesondere Oberflächen EMG-Signale ("surface EMG")-Signale (z.B. EMG Signale, die sich an einer Hautoberfläche ausbreiten), die auch als sEMG-Signale bezeichnet werden können, können durch andere Signale, z.B. überlagernde Spannungssignale, gestört werden. Dabei kann es sich dabei beispielsweise um eingekoppelte z.B. 50/60 Hz-Komponenten aus einem elektrischen Netz handeln. Ein weiteres Beispiel bei der Erfassung und/oder Detektion von EMG und/oder sEMG Signalen, beispielsweise in Herznähe, wären Überlagerungen mit dem Elektrokardiographie-Signal (EKG). Da sich die Frequenzbereiche von Nutzsignalen, beispielsweise EMG- und/oder sEMG-Signalen, und Störungen bzw. Störsignalen wie z.B. EKG-Signale oder andere, überlagern und/oder in einem ähnlichen Frequenzbereich liegen, müssen diese Komponenten, z.B. zur weiteren Auswertung, voneinander unterschieden bzw. getrennt werden.

Üblicherweise erfolgt die Separation mittels herkömmlicher Filtertechniken. Beispielsweise können zur Trennung von Nutz- und Störsignalen und/oder zur Unterdrückung der Störsignale analoge und digitale Frequenzfilter genutzt werden. Weitere Verfahren der digitalen Signalverarbeitung arbeiten beispielsweise mit komplexen adaptiven Wavelet-Filterbänken oder modell-basierten Ansätzen. Diese herkömmlichen Verfahren und/oder Filtertechniken sind jedoch nur bedingt zuverlässig bzw. akkurat, erfordern hohe Rechenkapazitäten und funktionieren nicht in Echtzeit. Ferner weisen sie hohe Latenzen auf.

Es ist Aufgabe der vorliegenden Erfindung, die Nachteile aus dem bekannten Stand der Technik zu überwinden und insbesondere ein verbessertes Verfahren zur Separation von Nutz- und Störsignalen in einer elektromyografischen, EMG, -Messung in einer elektrisch aktiven Region von Muskeln und/oder zur Unterdrückung von Störsignalen in der EMG-Messung bereitzustellen.

KURZBESCHREIBUNG DER ERFINDUNG

Die Aufgabe wird durch die Merkmale der unabhängigen Ansprüche gelöst. Die abhängigen Patentansprüche beschreiben bevorzugte Ausführungsformen. Weitere Aspekte, Vorteile und Merkmale ergeben sich aus den abhängigen Ansprüchen, der Beschreibung und den begleitenden Zeichnungen.

Gemäß **einem ersten Aspekt** betrifft die Erfindung ein Verfahren zur Separation von Nutz- und Störsignalen in einer elektromyografischen, EMG, -Messung in einer elektrisch aktiven Region von Muskeln und/oder zur Unterdrückung von Störsignalen in der EMG-Messung, das folgende Schritte umfasst:

- Erfassen eines ersten Signals mit wenigstens einer ersten Sensoreinrichtung auf einer ersten Muskelgruppe, wobei das erste Signal eine erste EMG-Signalkomponente und eine erste EMG-Störkomponente aufweist;
- Erfassen eines zweiten Signals mit wenigstens einer zweiten Sensoreinrichtung auf der ersten Muskelgruppe, wobei das zweite Signal eine zweite EMG-Signalkomponente und eine zweite EMG-Störkomponente aufweist;
- Ermitteln eines kombinierten Signals auf Basis des ersten Signals und des zweiten Signals zur Unterdrückung der ersten und/oder der zweiten EMG-Störkomponente.

Gemäß **einem zweiten Aspekt** betrifft die Erfindung die Verwendung des erfindungsgemäßen Verfahrens zum Monitoring von wenigstens einer Muskelgruppe, insbesondere Atemmuskeln, vorzugsweise zum Zwerchfell-Monitoring, und/oder zur Ansteuerung eines Beatmungsgerätes, und/oder zur Ansteuerung und/oder zum Monitoring von Prothesen und/oder Orthesen, und/oder zur Bewegungsanalyse.

Gemäß **einem dritten Aspekt** betrifft die Erfindung eine Vorrichtung zur Datenverarbeitung, umfassend Mittel zur Ausführung der Schritte des erfindungsgemäßen Verfahrens.

Gemäß **einem vierten Aspekt** betrifft die Erfindung ferner ein Computerprogrammprodukt, umfassend Befehle, die bei der Ausführung des Programms durch einen Computer diesen veranlassen, die Schritte des erfindungsgemäßen Verfahrens auszuführen.

Gemäß **einem fünften Aspekt** betrifft die Erfindung ferner ein Computerlesbares Speichermedium, umfassend Befehle, die bei der Ausführung durch einen Computer diesen veranlassen, die Schritte des erfindungsgemäßen Verfahrens auszuführen.

DETAILLIERTE BESCHREIBUNG DER ERFINDUNG

Im Folgenden werden die Elemente der Erfindung beschrieben. Diese Elemente sind mit spezifischen Ausführungsformen aufgeführt, wobei diese in beliebiger Weise und in beliebiger Anzahl kombiniert werden können, um zusätzliche Ausführungsformen zu schaffen. Die unterschiedlich beschriebenen Beispiele und bevorzugten Ausführungsformen sollten nicht so ausgelegt werden, dass sie die vorliegende Erfindung nur auf die explizit beschriebenen Ausführungsformen beschränken. Diese Beschreibung der Erfindung sollte so verstanden werden, dass sie Ausführungsformen unterstützt und umfasst, die zwei oder mehr der explizit beschriebenen Ausführungsformen kombinieren oder die eine oder mehrere der explizit beschriebenen Ausführungsformen mit einer beliebigen Anzahl der offenbarten und/oder bevorzugten Elemente kombinieren. Darüber hinaus sollten alle Permutationen und Kombinationen aller beschriebenen Elemente in dieser Anmeldung als durch die Beschreibung der vorliegenden Anmeldung offenbart betrachtet werden, sofern sich aus dem Kontext nichts anderes ergibt.

Gemäß **dem ersten Aspekt** betrifft die Erfindung ein Verfahren zur Separation von Nutz- und Störsignalen in einer elektromyografischen, EMG, -Messung in einer elektrisch aktiven Region von Muskeln und/oder zur Unterdrückung von Störsignalen in der EMG-Messung, das folgende Schritte umfasst:

- Erfassen eines ersten Signals mit wenigstens einer ersten Sensoreinrichtung auf einer ersten Muskelgruppe, wobei das erste Signal eine erste EMG-Signalkomponente und eine erste EMG-Störkomponente aufweist;
- Erfassen eines zweiten Signals mit wenigstens einer zweiten Sensoreinrichtung auf der ersten Muskelgruppe, wobei das zweite Signal eine zweite EMG-Signalkomponente und eine zweite EMG-Störkomponente aufweist;
- Ermitteln eines kombinierten Signals auf Basis des ersten Signals und des zweiten Signals zur Unterdrückung der ersten und/oder der zweiten EMG-Störkomponente.

Unter einer "elektromyografischen Messung" kann eine Messung von Muskelaktionspotentialen, z.B. elektrischen Signalen, die von Muskeln während ihrer Aktivität erzeugt werden, z.B. mittels differentieller Spannungsmessern, verstanden werden. Beispielsweise können die Muskelzellen während eines Muskelkontraktionsprozesses elektrische Aktivität in Form von Muskelaktionspotentialen erzeugen. Diese Potentiale können elektrische Ströme erzeugen, die sich über die Muskeln ausbreiten und durch Elektroden, z.B. auf und/oder unter der Haut, erfasst werden können. Durch die Messung der differentiellen Spannung zwischen den Elektroden können Informationen über die elektrische Aktivität des Muskels abgeleitet werden.

Ein "Störsignal" kann sich auf eine unerwünschte bzw. ungewollte Signalquelle und/oder -komponente beziehen, die in einem Messsystem, z.B. zwangsweise und/oder durchgehend, vorhanden ist und/oder eliminiert bzw. wenigstens reduziert werden soll. Dabei kann es sich, z.B. um ein elektrisches oder elektronisches, Signal handeln, welches sich auf das eigentliche, zu messende bzw. gewollte Nutzsignal auswirkt, insbesondere negativ, und z.B. dessen Qualität, Klarheit, Erkennbarkeit, und/oder Genauigkeit beeinträchtigen kann. Störsignale können verschiedene Ursachen haben, wie zum Beispiel elektromagnetische Interferenzen, Rauschen, Signalverzerrungen und/oder Überlagerungen von und/oder mit anderen Signalen.

Ein "Nutzsignal" hingegen kann sich auf ein gewünschtes Signal und/oder -komponente beziehen, das in einem Messsystem erfasst, gemessen, detektiert, und/oder ermittelt werden soll, z.B. zur Analyse, um daraus weitere Informationen zu gewinnen bzw. abzuleiten. Nutzsignale können beispielsweise von Störsignalen überlagert werden, wodurch eine Ermittlung und/oder Separation des Nutzsignals als solches erschwert werden kann.

Die Separation von Nutz- und Störsignalen kann sich auf den Prozess der (Ab-)Trennung, Unterscheidung, Separation und/oder Teilung des gewünschten Nutzsignals von den unerwünschten Störsignalen beziehen. Durch die Separation kann ein Anteil an Störsignalen eliminiert und/oder wenigstens reduziert werden, beispielsweise im Wesentlichen vollständig.

Unter einem ersten, zweiten, dritten, vierten etc. "Signal" kann eine physikalische Größe verstanden werden, die eine oder mehrere Informationen umfasst, z.B. eine Amplitude, die beispielsweise eine Stärke des Signals angibt, eine Frequenz, die beispielsweise eine Periodizität des Signals angibt, eine Phase, die beispielsweise eine zeitliche Verschiebung angibt, sowie eine Dauer und/oder eine Form. Das Signal kann auch eine oder mehrere Komponenten umfassen, beispielsweise ein Nutzsignal, insbesondere eine erste EMG-Signalkomponente und ein Störsignal, insbesondere eine erste EMG-Störkomponente aufweist.

Eine "EMG-Signalkomponente" kann sich auf die gewünschte elektrische Aktivität, die z.B. von den Muskeln einer ersten Muskelgruppe, erzeugt wird, beziehen und Informationen über die Muskelaktivität, wie z.B. die Kontraktion, Entspannung und/oder Bewegungsmuster o.ä. umfassen. Die EMG-Signalkomponente kann also als das Nutzsignal verstanden werden, das in der EMG-Messung erfasst und/oder analysiert werden soll. Diese könnte auch als EMG-Nutzkomponente oder EMG-Nutzsignalkomponente bezeichnet werden. Eine "EMG-Störkomponente" kann sich dagegen auf unerwünschte bzw. mit der EMG-Signalkomponente überlagerte Störsignale beziehen. Diese Störkomponente kann verschiedene Ursachen haben, wie zum Beispiel elektromagnetische Interferenzen, Rauschen aus der Umgebung, Signalverzerrungen durch die Elektrodenplatzierung, unerwünschte Muskelaktivitäten in benachbarten Muskeln und/oder andere Quellen, z.B. anderer Muskelgruppen in der Nähe. Ein Beispiel für eine Störkomponente ist ein Elektrokardiogramm (EKG)-Signal.

Ein Signal, dass von einer ersten Sensoreinrichtung erfasst wird, kann als erstes Signal bezeichnet werden und ein Signal, dass von einer zweiten Sensoreinrichtung erfasst wird, kann als zweites Signal bezeichnet werden, usw. Das erste Signal kann sich von dem zweiten Signal unterscheiden, beispielsweise dadurch, dass die erste Sensoreinrichtung das erste Signal an einer, z.B. örtlich, anderen Position misst, als die zweite Sensoreinrichtung. Die ersten und zweiten Signale können mittels der ersten Sensoreinrichtung und der zweiten Sensoreinrichtung z.B. jeweils örtlich aufgelöst, insbesondere (z.B. örtlich und/oder zeitlich) unabhängig voneinander, insbesondere differentiell erfasst werden, z.B. in einer bestimmten Richtung, Ausrichtung und/oder entlang einer bestimmten Achse.

Die erste Sensoreinrichtung und die zweite Sensoreinrichtung können bevorzugt auf einer, insbesondere gleichen, Muskelgruppe, beispielsweise einer ersten Muskelgruppe angebracht werden, vorzugsweise geometrisch oberhalb des betrachteten Muskels bzw. der betrachteten Muskelgruppe, z.B. auf einer darüberliegenden Hautoberfläche (z.B. nicht invasiv).

Ein erstes Signal kann eine erste EMG-Signalkomponente und eine erste EMG-Störkomponente aufweisen und ein zweites Signal kann eine zweite EMG-Signalkomponente und eine zweite EMG-Störkomponente aufweisen.

Die erste EMG-Signalkomponente kann sich von der zweiten EMG-Signalkomponente unterscheiden, insbesondere wenigstens teilweise. Beispielsweise kann sich die erste EMG-Signalkomponente von der zweiten EMG-Signalkomponente bezüglich eines, insbesondere zeitlichen, Signalverlaufs, insbesondere eines Spannungsverlaufs unterscheiden. Die erste EMG-Signalkomponente und die zweite EMG-Signalkomponente können jedoch einen im Wesentlichen identischen Informationsgehalt (z.B. gleicher Onset der Aktivität, gleiche Frequenz etc.), insbesondere einen im Wesentlichen identischen Informationsgehalt bezüglich einer myoelektrischen Aktivität umfassen, oder eine im Wesentlichen Identische Verteilung der myoelektrischen Aktivität der ersten Muskelgruppe umfassen. Mathematisch gesehen kann es sich bei der ersten EMG-Signalkomponente und der zweiten EMG-Signalkomponente um zwei statistisch unabhängige Messvariablen, z.B. mit der gleichen Verteilung handeln, die beispielsweise nicht korreliert sind. Dies wird beispielsweise dadurch erreicht, dass die erste EMG-Signalkomponente und die zweite EMG-Signalkomponente jeweils leicht unterschiedliche Anteile und/oder Muskelfasern, beispielsweise desselben Muskels messen, was beispielsweise aus der unabhängigen Positionierung und/oder Anordnung der ersten und zweiten Sensoreinrichtung folgen kann.

Unter einer "Sensoreinrichtung" kann ein Vorrichtung, Messaufbau, Detektor oder ein Gerät bezeichnet werden, das physikalische und/oder chemische Größen erfassen, detektieren, aufzeichnen, analysieren, erhalten, aufnehmen und/oder messen kann und/oder diese Größen in elektrische Signale umwandeln kann, z.B. zur Weitergabe und/oder zur weiteren Analyse. Eine Sensoreinrichtung, beispielsweise die erste Sensoreinrichtung und/oder die zweite Sensoreinrichtung, kann jeweils wenigstens zwei Elektroden umfassen, z.B. zur Bereitstellung eines ersten, insbesondere differentiellen, Messkanals und eines zweiten, insbesondere differentiellen, Messkanals, wobei die Elektroden Oberflächenelektroden und/oder Nadelelektroden sein können.

Die erste Sensoreinrichtung kann von der zweiten Sensoreinrichtung, z.B. auf einer ersten Muskelgruppe beanstandet angeordnet sein, wobei der Abstand von einer Art und/oder eine Größe der ersten Muskelgruppe abhängen kann. Beispielsweise lässt eine große Muskelgruppe, wie z.B. das Zwerchfell, z.B. bei einem Erwachsenen, einen Abstand von ca. 30 cm (oder weniger/mehr) zu. Eine andere Muskelgruppe, z.B. in der Schulter, z.B. bei Kindern, kann hingegen nur einen Abstand von ca. 5 cm (oder weniger/mehr) zulassen. Der Abstand kann also von der Anatomie, von der Art der Muskelgruppe, den Gegebenheiten auf der Hautoberfläche und/oder von der untersuchten Person (z.B. einem Subjekt) abhängen (z.B. Größe, Alter, etc.).

Gemäß dem erfindungsgemäßen Verfahren wird ein "kombiniertes Signal" Signals auf Basis des ersten Signals und des zweiten Signals ermittelt, insbesondere zur Unterdrückung der ersten und/oder der zweiten EMG-Störkomponente. Ein "kombiniertes Signal" kann als eine Kombination, ein Zusammenführen und/oder eine Überlagerung des ersten Signals mit dem zweiten Signal verstanden werden, oder umgekehrt. Insbesondere kann das Ermitteln eine Separation der ersten und/oder der zweiten EMG-Störkomponente umfassen, wobei die Separation auf einer Subtraktion des ersten und des zweiten Signals basiert. Beispielsweise kann das erste Signal von dem zweiten Signal subtrahiert, abgezogen und/oder entfernt werden, oder umgekehrt, um das kombinierte Signal zu erhalten bzw. zu ermitteln. Insbesondere kann durch eine Subtraktion des ersten von dem zweiten Signal, oder umgekehrt, sowohl die ersten als auch die zweite EMG-Störkomponente im Wesentlichen verschwinden, beispielsweise da diese im Wesentlichen identisch sind.

Das erfindungsgemäße Verfahren kann optional, Visualisieren des kombinierten Signals (oder auch mehrerer), insbesondere eines Ursprungs des kombinierten Signals (oder auch mehrerer) umfassen, wobei das Visualisieren insbesondere ein räumliches und/oder zeitliches Visualisieren mittels einer Heatmap sein kann. Mittels einer Heatmap können z.B. Daten, Informationen, Signale etc. auf einer Karte und/oder einem räumlichen Diagramm dargestellt werden, wobei verschiedene Werte, Größen und/oder Intensitäten durch Farben visualisiert werden können. Die Daten, Informationen, Signale und/oder Bereiche können auf der Karte anhand einer Farbskala dargestellt werden, wobei beispielsweise höhere Werte und/oder Intensitäten beispielsweise durch wärmere Farben wie Rot oder Orange repräsentiert werden und niedrigere Werte beispielsweise durch kühlere Farben wie Blau oder Grün repräsentiert werden können. Eine Heatmap kann es ermöglichen, räumliche und/oder zeitliche Muster, Verläufe, Trends, Bereich und/oder Unterschiede in den Daten, Informationen, Signalen darzustellen bzw. zu erkennen.

Das erfindungsgemäße Verfahren kann ferner umfassen: Anpassen von Signalamplituden, insbesondere Spannungsamplituden der ersten EMG-Störkomponente und Signalamplituden, insbesondere Spannungsamplituden, der zweiten EMG-Störkomponente, insbesondere derart, dass die Signalamplituden, insbesondere die Spannungsamplituden, der ersten EMG-Störkomponente im Wesentlichen den Signalamplituden, insbesondere den Spannungsamplituden, der zweiten EMG-Störkomponente entsprechen. Wie oben bereits beschrieben, sind die erste EMG-Störkomponente und die zweite EMG-Störkomponente im Wesentlichen identisch. Kleinere Abweichungen, Unterschiede und/oder Diskrepanzen können jedoch mittels der Anpassung ausgeglichen werden, z.B. um sicherzustellen, dass die EMG-Störkomponenten bei einer Kombination der Signale, z.B. bei einer Subtraktion, im Wesentlichen verschwinden.

Die Anpassung bzw. das Anpassen kann auch als eine Normierung bezeichnet werden. Die Normierung kann z.B. über verschiedene Rechenvorschriften erfolgen darunter z.B. durch Bilden des Verhältnisses der maximalen Signalamplituden der EMG-Störkomponenten und/oder allgemein durch statistische Schätzverfahren wie z.B. Regression und/oder durch adaptive Filter. Durch die Anpassung kann erreicht werden, dass die erste EMG-Störkomponente im Wesentlichen der zweiten EMG-Störkomponente entspricht, wodurch diese, z.B. beim Ermitteln des kombinierten Signals, verschwinden, beispielsweise dadurch, dass deren Werte, insbesondere deren Amplituden, genauer aufeinander abgestimmt sind. Hierdurch kann die z.B. die Genauigkeit verbessert und/oder das resultierende bzw. kombinierte Signal deutlicher ermittelt werden. Das Anpassen ist jedoch optional.

Das Anpassen kann dynamisch und/oder kontinuierlich erfolgen (auch mehrfach, bzw. widerholt), insbesondere während des Erfassens des ersten und des zweiten Signals und/oder zeitlich vor dem Ermitteln des kombinierten Signals.

Die erste EMG-Störkomponente und/oder die zweite EMG-Störkomponente können ein Elektrokardiogramm, EKG, -Signal, insbesondere ein EKG-Spannungssignal, umfassen. Die erste EMG-Störkomponente und/oder die zweite EMG-Störkomponente können von einer gleichen Quelle verursacht werden und/oder davon ausgehen, z.B. von einem Herzmuskel. Dies kann auch als erste Quelle bezeichnet werden. Demgegenüber können die EMG-Signalkomponenten, die beispielsweise auf einer ersten Muskelgruppe erfasst werden, als zwei, insbesondere verschiedene, bzw. unabhängige Quellen aufgefasst werden. Dies kann beispielsweise aus der unabhängigen Positionierung und/oder Anordnung der ersten und zweiten Sensoreinrichtung auf der ersten Muskelgruppe, z.B. geometrisch darüber auf der Haut (z.B. nicht invasiv), resultieren.

Das erfindungsgemäße Verfahren kann ferner umfassen: Anbringen der ersten Sensoreinrichtung und/oder der zweiten Sensoreinrichtung

-- auf unterschiedlichen Positionen der ersten Muskelgruppe, insbesondere benachbart, wobei die erste Muskelgruppe insbesondere Atemmuskeln umfasst, vorzugsweise ein Zwerchfell; und/oder
-- in einem im Wesentlichen identischen Abstand zu einer zweiten Muskelgruppe, wobei sich die zweite Muskelgruppe von der ersten Muskelgruppe unterscheidet, wobei die zweite Muskelgruppe insbesondere Herzmuskeln umfasst.

Auf unterschiedlichen Positionen und/oder benachbart kann beispielsweise äquidistant bedeuten. Beispielsweise können insbesondere die Elektroden der ersten Sensoranordnung und die Elektroden der zweiten Sensoranordnung in gleichen Abständen zueinander angebracht werden, z.B. einem Abstand von 1 cm zueinander oder auch mehr bzw. weniger. Werden mehrere Sensoranordnungen verwendet, können diese auch in jeweils gleichen Abständen zueinander angeordnet werden, beispielsweise in einem Abstand zueinander, der von der Art und/oder einer Größe der Muskelgruppe abhängen kann, wie oben beschrieben.

Unter einem "im Wesentlichen identischen Abstand zu einer zweiten Muskelgruppe" kann verstanden werden, dass die erste und die zweite Sensoreinrichtung so angeordnet werden, das ein Abstand bzw. eine Distanz zur zweiten Muskelgruppe im Wesentlichen gleich bzw. identisch ist und/oder ein Abstand der Sensoreinrichtungen zueinander im Verhältnis dazu vernachlässigbar ist. Beispielsweise können die erste Sensoreinrichtung und/oder die zweite Sensoreinrichtung derart in Bezug zueinander angeordnet, platziert und/oder angebracht werden, dass diese eine Achse definieren, wobei die Achse im Wesentlichen in Richtung der zweiten Muskelgruppe zeigt, z.B. also so, dass die Sensoreinrichtungen, in Richtung des Herzmuskels zeigend, untereinander, hintereinander und/oder nacheinander angeordnet bzw. angebracht sind. Auch wenn sich die erste Sensoreinrichtung in diesem Beispiel auf dieser Achse, dann oberhalb, z.B. näher, an dem Herzmuskel als die zweite Sensoreinrichtung befindet, oder umgekehrt, kann dies als "im Wesentlichen identischer Abstand" verstanden werden, da ein Abstand der Sensoreinrichtungen zueinander im Vergleich bzw. Verhältnis zur Strecke, und/oder Distanz zu der zweiten Muskelgruppe, z.B. zu dem Herzmuskel, entsprechend kleiner und daher vernachlässigbar ist. Anzumerken ist jedoch, dass die Anordnung entlang einer bestimmten Achse, z.B. in Richtung des Herzmuskels zeigend, lediglich eine bevorzugte Anordnung ist, d.h. optional ist, da sich bei einer derartigen Anordnung besonders gute Ergebnisse/Messungen erzielen lassen. Auch andere Anordnungen sind daher möglich und führen ebenfalls zu guten Ergebnissen.

Das erfindungsgemäße Verfahren kann mittels zwei hierin beschriebenen Sensoreinrichtungen realisiert werden, die entsprechend jeweils zwei Elektroden umfassen können. Optional können jedoch auch mehrere Sensoreinrichtungen verwendet werden und diese, z.B. auf mehreren Muskeln und/oder mehreren Stellen auf dem gleichen Muskel angebracht werden. Beispielsweise können die erste Sensoreinrichtung und die zweite Sensoreinrichtung kopiert und/oder dupliziert werden. Damit kann beispielsweise eine räumliche und/oder zeitliche Auflösung, z.B. 2D oder 3D, der Aktivität, insbesondere der Muskelaktivität, erhalten werden. Dies kann ebenfalls mit einer hierin beschriebenen Heatmap visualisiert werden, z.B. als Animation, um eine Ausbreitung und/oder eine Ausbreitungsrichtung der Aktivität darzustellen.

Das erfindungsgemäße Verfahren kann ferner umfassen:

Bereitstellen einer duplizierten Sensoranordnung, die eine dritte Sensoreinrichtung zum Erfassen eines dritten Signals und/oder der vierte Sensoreinrichtung zum Erfassen eines vierten Signals umfasst;
Anbringen der duplizierten Sensoranordnung auf der ersten Muskelgruppe und/oder wenigstens einer weiteren Muskelgruppe, die sich von der ersten Muskelgruppe unterscheidet.

Wie zuvor erwähnt kann die erste Muskelgruppe beispielsweise ein Zwerchfell sein, und die dritte Muskelgruppe kann beispielsweise ein Schultermuskel sein.

Unter "dupliziert" kann eine Wiederholung, ein Kopieren und/oder eine Duplikation der Elemente, Komponenten und/oder Teile, z.B. der ersten und/oder zweiten Sensoreinrichtungen und/oder der in diesem Zusammenhang offenbarten Verfahrensschritte verstanden werden, beispielsweise, dass diese in gleicher Weise für die dritte und vierte Sensoranordnung wiederholt werden können. Es sei daher klar, dass das hierin bezüglich der ersten/zweiten Sensoreinrichtungen und/oder des erfindungsgemäßen Verfahrens gesagte sich gleichermaßen auf die dritte und vierte Sensoranordnung, sowie die diesbezüglich beschriebenen Verfahrensschritte bezieht und umgekehrt.

Eine "duplizierte Sensoranordnung" kann also eine Kopie bzw. ein Duplikat der ersten und zweiten Sensoreinrichtungen darstellen und eine dritte Sensoreinrichtung zum Erfassen eines dritten Signals und/oder der vierte Sensoreinrichtung zum Erfassen eines vierten Signals umfassen.

Das erfindungsgemäße Verfahren kann ferner umfassen: Ermitteln eines weiteren kombinierten Signals auf Basis des dritten Signals und des vierten Signals. Das erfindungsgemäße Verfahren kann ferner umfassen: Visualisieren des kombinierten Signals und des weiteren kombinierten Signals, insbesondere mittels einer Heatmap, um eine räumliche und/oder zeitliche Auflösung zu erhalten.

Gemäß **dem zweiten Aspekt** betrifft die Erfindung die Verwendung des erfindungsgemäßen Verfahrens zum Monitoring von wenigstens einer Muskelgruppe, insbesondere Atemmuskeln, vorzugsweise zum Zwerchfell-Monitoring, und/oder zur Ansteuerung eines Beatmungsgerätes, und/oder zur Ansteuerung und/oder zum Monitoring von Prothesen und/oder Orthesen, und/oder zur Bewegungsanalyse.

Mögliche Verwendungen und/oder Anwendungen des erfindungsgemäßen Verfahrens umfassen eine oder mehrere von: sportmedizinische Anwendungen, z.B. bei der Schulter, darunter z.B. die Untersuchung der Trainingsintensität und/oder Erkennung von Langzeittrends im Training. Darauf basierend könnten z.B. Über-/Unteranstrengung erkannt werden, wobei Muskelfatigue nach und/oder während des Trainings z.B. durch das EMG-Frequenzspektrum detektiert werden kann. Auch Anwendungen in Richtung Biofeedback wäre denkbar, z.B. eine Visualisierung der Trainingsintensität für den Sportler in Echtzeit und somit eine direkte Anpassung der Muskelaktivität (z.B. bei einer Kraftübung). Eine weitere Verwendung und/oder Anwendung wäre in der Prothetik z.B. die direkte Ansteuerung von Handprothesen. Hier könnte das EMG des Unterarms für eine direkte Ansteuerung der Prothese eingesetzt werden. Beispielsweise könnte eine bestimmte EMG-Aktivierung z.B. eine Greifbewegung der Prothese auslösen etc.

Gemäß **dem dritten Aspekt** betrifft die Erfindung eine Vorrichtung zur Datenverarbeitung, umfassend Mittel zur Ausführung der Schritte des erfindungsgemäßen Verfahrens.

Gemäß **dem vierten Aspekt** betrifft die Erfindung ferner ein Computerprogrammprodukt, umfassend Befehle, die bei der Ausführung des Programms durch einen Computer diesen veranlassen, die Schritte des erfindungsgemäßen Verfahrens auszuführen.

Gemäß **dem fünften Aspekt** betrifft die Erfindung ferner ein Computerlesbares Speichermedium, umfassend Befehle, die bei der Ausführung durch einen Computer diesen veranlassen, die Schritte des erfindungsgemäßen Verfahrens auszuführen.

Die Erfindung betrifft außerdem die nachfolgend beschriebenen besonderen Ausführungsformen, welche mit allen Aspekten und Ausführungsformen der Erfindung beliebig kombiniert werden können.

Wie oben bereits beschrieben kann das erfindungsgemäße Verfahren ein Anpassen von Signalamplituden, insbesondere Spannungsamplituden, der ersten EMG-Störkomponente und Signalamplituden, insbesondere Spannungsamplituden, der zweiten EMG-Störkomponente, umfassen. Wie oben ebenfalls beschrieben, kann die Anpassung bzw. das Anpassen auch als eine Normierung bezeichnet werden. Die Normierung kann z.B. über verschiedene Rechenvorschriften erfolgen darunter z.B. durch Bilden des Verhältnisses der maximalen Signalamplituden der EMG-Störkomponenten und/oder allgemein durch statistische Schätzverfahren wie z.B. Regression und/oder durch adaptive Filter. In einer besonderen bevorzugten Ausführungsform umfasst die Regression einen gewöhnliche Kleinste-Quadrat-Schätzung. Durch diesen Schritt kann der Grad, zu welchem sich die Signalamplituden der ersten EMG-Störkomponente und der zweiten EMG-Störkomponente entsprechen, erhöht werden. Dadurch kann die Genauigkeit des erfindungsgemäßen Verfahrens noch weiter verbessert und/oder das resultierende bzw. kombinierte Signal noch deutlicher ermittelt werden.

Wie oben bereits beschrieben, kann die Anpassung dynamisch und/oder kontinuierlich erfolgen. In einer besonderen bevorzugten Ausführungsform umfasst die dynamische Anpassung ein zeitlich adaptives rechnerisches und/oder statistisches Verfahren. Vorzugsweise ist dieses Verfahren ein rekursives Verfahren, z.B. eine Regression über Recursive Least Squares oder eine Systemidentifikation. Durch diese dynamische Anpassung kann die Amplitude der ersten EMG-Störkomponente und/oder zweiten EMG-Störkomponente optimal angepasst werden.

In bevorzugten Ausführungsformen umfasst das erfindungsgemäße Verfahren ein Bereitstellen einer duplizierten Sensoranordnung, die eine dritte Sensoreinrichtung zum Erfassen eines dritten Signals und/oder der vierte Sensoreinrichtung zum Erfassen eines vierten Signals umfasst; und Anbringen der duplizierten Sensoranordnung auf der ersten Muskelgruppe und/oder wenigstens einer weiteren Muskelgruppe, die sich von der ersten Muskelgruppe unterscheidet.

In einer besonderen bevorzugten Ausführungsform umfasst das erfindungsgemäße Verfahren ein Anbringen der duplizierten Sensoreinrichtung auf einem Zwerchfell der rechten Körperseite und/oder auf einem Zwerchfell der linken Körperseite. In dieser besonderen bevorzugten Ausführungsform kann eine erste und eine zweite Sensoreinrichtung auf einem Zwerchfell der rechten Körperseite und eine dritte und eine vierte Sensoreinrichtung auf einem Zwerchfell der linken Körperseite angebracht sein. Alternativ kann in dieser besonderen bevorzugten Ausführungsform eine erste und eine zweite Sensoreinrichtung auf einem Zwerchfell der linken Körperseite und eine dritte und eine vierte Sensoreinrichtung auf einem Zwerchfell der rechten Körperseite angebracht sein. Die damit bestimmten EMG-Signalkomponenten ermöglichen Unterschiede in der Aktivität der linken und rechten Zwerchfell-Kuppel festzustellen, wodurch beispielsweise eine einseitige Zwerchfellparese diagnostiziert werden kann.

Die hierin verwendeten Begriffe "nach der [vorliegenden] Erfindung", "erfindungsgemäß" und dergleichen sollen sich auf alle beschriebenen Aspekte und Ausführungsformen der Erfindung beziehen, die hierin beschrieben und/oder beansprucht werden.

Der Begriff "umfassend", wie er hierin verwendet wird, ist so auszulegen, dass er sowohl "einschließlich" als auch "bestehend aus" umfasst, wobei beide Bedeutungen spezifisch beabsichtigt sind und somit individuell offenbarte Ausführungsformen gemäß der vorliegenden Erfindung offenbart werden. Sofern hierin die Bezeichnung "und/oder" verwendet wird, ist die Bezeichnung "und/oder" als spezifische Offenbarung jedes der beiden spezifizierten Merkmale oder Komponenten mit oder ohne das andere zu verstehen. Zum Beispiel ist "A und/oder B" als spezifische Offenbarung von jedem von (i) A, (ii) B und (iii) A und B zu verstehen, so als ob jedes hier einzeln aufgeführt wäre. Im Zusammenhang mit der vorliegenden Erfindung bezeichnen die Begriffe "ungefähr" bzw. "etwa" ein Genauigkeitsintervall, welches der Fachmann versteht, um die technische Wirkung des betreffenden Merkmals dennoch sicherzustellen. Der Begriff gibt typischerweise eine Abweichung vom angegebenen Zahlenwert um $\pm 20\%$, $\pm 15\%$, $\pm 10\%$ und beispielsweise $\pm 5\%$ an. Wie der Durchschnittsfachmann erkennen wird, hängt die spezifische derartige Abweichung für einen numerischen Wert für einen gegebenen technischen Effekt von der Art des technischen Effekts ab. Beispielsweise kann ein natürlicher oder biologischer technischer Effekt im Allgemeinen eine größere solche Abweichung aufweisen als ein von Menschen verursachter Effekt. Sofern ein unbestimmter oder bestimmter Artikel verwendet wird, wenn man sich auf ein Nomen im Singular bezieht, z.B. "ein" oder "eine", so schließt dies den Plural dieses Nomens ein, es sei denn, es ist hierin etwas anderes ausdrücklich angegeben.

Sofern der Kontext nichts anderes vorschreibt, sind die Beschreibungen und Definitionen der oben dargelegten Merkmale nicht auf einen bestimmten Aspekt oder eine bestimmte Ausführungsform der Erfindung beschränkt und gelten gleichermaßen für alle beschriebenen Aspekte und Ausführungsformen der Erfindung.

Die Anwendung der Lehren der vorliegenden Erfindung auf ein bestimmtes Problem oder Umfeld und die Einbeziehung von Variationen der vorliegenden Erfindung oder zusätzlichen Merkmalen dazu (wie weitere Aspekte und Ausführungsformen), liegt im Bereich der Möglichkeiten einer Person mit durchschnittlichen Fähigkeiten im Fachgebiet im Einklang mit den hier enthaltenen Lehren der Erfindung.

Alle hier zitierten Referenzen, Patente, Patentveröffentlichungen und sonstige Veröffentlichungen werden hiermit in ihrer Gesamtheit durch Bezugnahme in diese Offenbarung aufgenommen.

KURZE BESCHREIBUNG DER FIGUREN

Ausführungsbeispiele der Erfindung werden nun unter Bezugnahme auf die beigefügten Zeichnungen beschrieben. Damit die oben genannten Merkmale der vorliegenden Offenbarung im Detail verstanden werden können, kann eine detailliertere Beschreibung der Offenbarung, die oben kurz zusammengefasst wurde, durch Bezugnahme auf Ausführungsbeispiele erhalten werden. Die begleitenden Zeichnungen beziehen sich auf Ausführungsformen der Offenbarung und werden im Folgenden beschrieben:

Fig. 1    zeigt eine erfindungsgemäße Anordnung einer ersten und zweiten Sensoreinrichtung, die auf einer ersten Muskelgruppe angebracht sind, mittels derer das erfindungsgemäße Verfahren durchgeführt werden kann;

Fig. 2    zeigt beispielhafte Messsignale der erfindungsgemäßen Anordnung aus Fig. 1, insbesondere ein erstes Signal, das mit der ersten Sensoreinrichtung erfasst wird und ein zweites Signal, das mit der zweiten Sensoreinrichtung erfasst wird; und

Fig. 3    zeigt ein beispielhaftes kombiniertes Signal auf Basis des erfassten ersten Signals und des zweiten Signals aus Fig. 2.

Im Folgenden soll die Erfindung anhand von in den Zeichnungen dargestellten Ausführungsformen näher erläutert werden, wobei in sämtlichen Zeichnungen im Wesentlichen funktionsgleiche Elemente die gleichen Bezugszeichen haben.

Die Zeichnungen sind schematische Zeichnungen, die nicht maßstabsgetreu sind. Einige Elemente in den Zeichnungen können übertriebene Abmessungen aufweisen, um Aspekte der vorliegenden Offenbarung zu betonen und/oder für eine bessere Präsentationsklarheit. Zur Vereinfachung werden identische Bezugszeichen verwendet, um identische Elemente zu kennzeichnen, die in den Zeichnungen gemeinsam enthalten sind. Es ist vorgesehen, dass Elemente und Merkmale einer Ausführungsform vorteilhaft in andere Ausführungsformen ohne weitere Erwähnung eingebaut werden können. Im Allgemeinen werden nur die Unterschiede bezüglich individueller Ausführungsformen beschrieben.

Jedes Ausführungsbeispiel dient zur Erläuterung der Offenbarung und soll nicht als Beschränkung der Offenbarung verstanden werden. Darüber hinaus können Merkmale, die als Teil einer Ausführungsform dargestellt oder beschrieben werden, in Verbindung mit anderen Ausführungsformen verwendet werden, um eine weitere Ausführungsform zu erzeugen. Es ist beabsichtigt, dass die Beschreibung solche Modifikationen und Variationen einschließt.

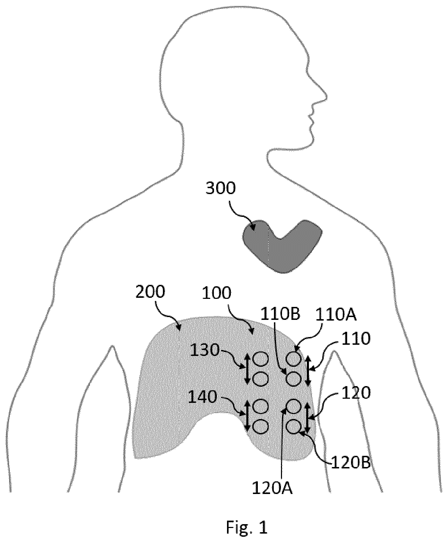

Fig. 1 zeigt eine erfindungsgemäße Anordnung 100 einer ersten Sensoreinrichtung 110, die eine erste Elektrode 110A und eine zweite Elektrode 110B umfassen kann, sowie einer und zweiten Sensoreinrichtung 120, die ebenfalls eine erste Elektrode 120A und eine zweite Elektrode 120B umfassen kann. Die erste Sensoreinrichtung 110 und die zweite Sensoreinrichtung 120 sind in Fig. 1 durch einen Pfeil dargestellt, welcher auch als Messkanal, bzw. erster Messkanal 110 und zweiter Messkanal 120, bezeichnet werden kann. Wie in Fig. 1 dargestellt können die erste Sensoreinrichtung 110 und die zweite Sensoreinrichtung 120 auf einer ersten Muskelgruppe 200, beispielsweise einem Zwerchfell 200, angebracht sein, z.B. geometrische und/oder örtlich darüber, beispielsweise auf der Haut (z.B. nicht invasiv).

Optional kann eine duplizierte Sensoranordnung 130, 140 bereitgestellt werden, die eine dritte Sensoreinrichtung 130 zum Erfassen eines dritten Signals und/oder der vierte Sensoreinrichtung 140 zum Erfassen eines vierten Signals umfasst. In Fig. 1 ist die duplizierte Sensoranordnung 130, 140 ebenfalls auf der ersten Muskelgruppe 200 angebracht, beispielsweise beabstandet von der ersten Sensoreinrichtung 110 und der zweiten Sensoreinrichtung 120, wobei dieser Abstand von einer Art und/oder Größe des Muskels abhängen kann. Alternativ oder zusätzlich kann die duplizierte Sensoranordnung 130, 140 jedoch auch auf wenigstens einer weiteren Muskelgruppe (nicht gezeigt) angebracht werden, die sich von der ersten Muskelgruppe 200 unterscheidet. Die dritte Sensoreinrichtung 130 und/oder die vierte Sensoreinrichtung 140 können auch jeweils zwei Elektroden 130A-B, 140 AB umfassen, welche aus Übersichtlichkeitsgründen in Fig. 1 nicht mit Bezugszeichen versehen sind. Anzumerken ist, dass die duplizierte Sensoranordnung, insbesondere die dritte und vierte Sensoreinrichtung 130, 140 optional sind.

Wie in Fig. 1 dargestellt, sind die erste Sensoreinrichtung 110 und/oder die zweite Sensoreinrichtung 120 derart in Bezug zueinander angeordnet, platziert und/oder angebracht, dass diese eine Achse definieren, wobei die Achse im Wesentlichen in Richtung der zweiten Muskelgruppe 300 zeigt, also z.B. in Richtung des Herzmuskels. Mit anderen Worten, die erste Sensoreinrichtung 110 und die zweite Sensoreinrichtung 120 sind in einer Linie in Richtung des Herzens 300 angeordnet, also, wie in Fig. 1 beispielshaft gezeigt, sind die erste Sensoreinrichtung 110 und/ die zweite Sensoreinrichtung 120 untereinander, hintereinander und/oder nacheinander angeordnet bzw. angebracht. Wie zu sehen ist, kann sich die erste Sensoreinrichtung 110 damit näher an dem Herzmuskel 300 befinden als die zweite Sensoreinrichtung 120. Der Abstand der ersten und zweiten Sensoreinrichtung 110, 120 zu dem Herzmuskel ist jedoch im Vergleich zu dem Abstand der ersten und zweiten Sensoreinrichtung 110, 120 zueinander im Vergleich bzw. Verhältnis dazu kleiner, wodurch dieser vernachlässigbar wird. Dadurch können die Sensoreinrichtungen 110, 120 einen im Wesentlichen identischen Abstand zum Herzen aufweisen, bzw. dies so definiert und/oder festgelget werden.

Mit der in Fig. 1 gezeigten Anordnung kann nun beispielsweise eine Zwerchfellaktivität, eines Zwerchfells 200, erfasst werden, wobei auch andere Muskeln und/oder Muskelgruppen erfasst werden können. Die erste Sensoreinrichtung 110 kann also einen ersten Messkanal 110, z.B. an einer ersten Stelle oder Position, darstellen und die zweite Sensoreinrichtung 120 einen zweiten Messkanal 120, z.B. an einer zweiten, insbesondere anderen und/oder davon unabhängigen, Stelle oder Position. Wie zuvor beschrieben, kann die hohe örtliche Sensitivität des EMG-Signals dazu führen, dass die Signalverläufe, insbesondere der zeitlichen Signalverläufe der EMG-Signalkomponenten, z.B. der ersten EMG-Signalkomponente, die mittels der ersten Sensoreinrichtung 110 erfasst wird und der zweiten EMG-Signalkomponente, die mittels der zweiten Sensoreinrichtung 120 erfasst wird unterschiedlich sind; jedoch aber einen im Wesentlichen identischen Informationsgehalt, insbesondere einen im Wesentlichen identischen Informationsgehalt bezüglich einer myoelektrischen Aktivität umfassen, oder eine im Wesentlichen Identische Verteilung der myoelektrischen Aktivität der ersten Muskelgruppe umfassen, z.B. also die gleichen Informationen hinsichtlich der Muskelkontraktion, z.B. des Zwerchfells 200 bei einem Atemvorgang, umfassen.

Aufgrund der zuvor beschriebenen großen, jedoch fast identischen Entfernung, bzw. dem Abstand, in Richtung zum Herzen 300 werden, die erste Sensoreinrichtung 110 und die zweite Sensoreinrichtung 120 jeweils von einem im Wesentlichen identischen EMG-Störkomponente, insbesondere einem EKG-Signal und/oder einem EKG-Signalanteil EKG überlagert.

Mathematisch gesehen kann dies wie folgt ausgedrückt werden:

$$CH_{1a} = EMG_{1a} + EKG; \qquad CH_{1b} = EMG_{1b} + EKG; (1)$$

wobei $CH_{1a}$ für das erste Signal stehen kann, das mit dem ersten Messkanal 110 ("channel") bzw. der erste Sensoreinrichtung 110 erfasst werden kann und $CH_{1b}$ für das zweite Signal, das mit dem zweiten Messkanal 120 ("channel") bzw. der zweite Sensoreinrichtung 120 erfasst werden kann. Das erste Signal kann eine erste EMG-Signalkomponente $EMG_{1a}$ umfassen und das zweite Signal eine zweite EMG-Signalkomponente $EMG_{1b}$.

Wie durch die Indizes $_{1a}$ und $_{1b}$ angedeutet, kann sich erste EMG-Signalkomponente $EMG_{1a}$ von der zweiten EMG-Signalkomponente $EMG_{1b}$ unterscheiden, insbesondere wenigstens teilweise. Beispielsweise kann sich die erste EMG-Signalkomponente $EMG_{1a}$ von der zweiten EMG-Signalkomponente $EMG_{1b}$ bezüglich eines, insbesondere zeitlichen, Signalverlaufs, insbesondere eines Spannungsverlaufs unterscheiden. Die erste EMG-Signalkomponente $EMG_{1a}$ und die zweite EMG-Signalkomponente $EMG_{1b}$ können jedoch einen im Wesentlichen identischen Informationsgehalt (z.B. gleicher Onset der Aktivität, gleiche Frequenz etc.), insbesondere einen im Wesentlichen identischen Informationsgehalt bezüglich einer myoelektrischen Aktivität umfassen, oder eine im Wesentlichen Identische Verteilung der myoelektrischen Aktivität der ersten Muskelgruppe umfassen. Mathematisch gesehen kann es sich bei der ersten EMG-Signalkomponente $EMG_{1a}$ und der zweiten EMG-Signalkomponente $EMG_{1b}$ um zwei statistisch unabhängige Messvariablen, z.B. mit der gleichen Verteilung handeln, die beispielsweise nicht korreliert sind. Dies wird beispielsweise dadurch erreicht, dass die erste EMG-Signalkomponente und die zweite EMG-Signalkomponente jeweils leicht unterschiedliche Anteile und/oder Muskelfasern, beispielsweise desselben Muskels messen, was beispielsweise aus der unabhängigen Positionierung und/oder Anordnung der ersten und zweiten Sensoreinrichtung 110, 120 folgen kann.

Wie weiter aus Formel (1) hervorgeht, kann EKG für die erste EMG-Störkomponente und/oder die zweite EMG-Störkomponente stehe, welche in diesem Beispiel ein Elektrokardiogramm, EKG, -Signal, insbesondere ein EKG-Spannungssignal, umfasst, wobei insbesondere die erste EMG-Störkomponente und/oder die zweite EMG-Störkomponente von einer gleichen Quelle verursacht werden und/oder ausgehen, insbesondere von dem Herzmuskel 300. Wie in Formel 1 dargestellt, sind die erste EMG-Störkomponente und die zweite EMG-Störkomponente im Wesentlichen identisch, wobei zusätzlich und optional eine hierin beschriebene Anpassung bzw. Normierung angewendet werden kann, beispielsweise um die EMG-Störkomponenten genauer aufeinander abzustimmen.

Erfindungsgemäß wird auf Basis des ersten Signals $CH_{1a}$ und des zweiten Signals $CH_{1b}$ ein Kombiniertes Signal $CH_k$ ermittelt, beispielsweise zur Unterdrückung der ersten und/oder der zweiten EMG-Störkomponente (z.B. EKG). Mathematisch gesehen kann dies beispielsweise durch eine Subtraktion erreicht werden:

$$CH_k = CH_{1b} - CH_{1a} = (EMG_{1b} + EKG) - (EMG_{1a} + EKG) \qquad (2a)$$

wobei $CH_k$ für das ermittelte kombinierte Signal stehen kann. Es sei klar, dass alternativ auch die Rechenvorschrift $CH_{1a} - CH_{1b}$ angewendet werden könnte.

Es sei auch klar, dass bei Verwendung einer duplizierten Sensoranordnung ein weiteres kombiniertes Signal $CH_{k2}$ die folgende Form haben kann:

$$CH_{k2} = CH_{2b} - CH_{2a} = (EMG_{2b} + EKG) - (EMG_{2a} + EKG) \qquad (2b)$$

wobei $CH_{2a}$ für das dritte Signal stehen kann, das mit dem dritten Messkanal 130 ("channel") bzw. der dritten Sensoreinrichtung 130 erfasst werden kann und $CH_{2b}$ für das vierte Signal, das mit dem vierten Messkanal 140 ("channel") bzw. der vierten Sensoreinrichtung 140 erfasst werden kann. Das dritte Signal kann eine dritte EMG-Signalkomponente $EMG_{2a}$ umfassen und das vierte Signal eine vierte EMG-Signalkomponente $EMG_{2b}$ usw. Aus diesen Signalen kann sodann ein weiteres kombiniertes Signal $CH_{k2}$ im Sinne der Erfindung, z.B. durch Subtraktion, ermittelt werden. Hierbei gilt, wie schon erwähnt, das bezüglich des erfindungsgemäßen Verfahrens und/oder das bezüglich der ersten und zweiten Sensoreinrichtung 110, 120 gesagte.

Wie bereits erwähnt, kann es z.B. je nach Messanordnung, sinnvoll sein, vor der Subtraktion eine (dynamische) Normierung der Messkanäle vorzunehmen, sodass die auftretenden EKG-Signalanteile tatsächlich, z.B. die gleiche Amplitude aufweisen. Dadurch, dass die EMG-Störkomponenten des ersten Signals und des zweiten Signals im Wesentlichen identisch sind, ergibt sich das folgende kombinierte Signal:

$$CH_k = EMG_{1b} - EMG_{1a} \qquad (3)$$

In dem kombinierten Signal kann die EKG-Störkomponente also unterdrückt werden, wodurch das Signal $CH_k$ lediglich die EMG-Signalkomponenten $EMG_{1a}$ und $EMG_{1b}$ umfasst. Dadurch können Nutz- und Störsignalen in der EMG, -Messung in einer elektrisch aktiven Region von Muskeln, beispielsweis an dem Zwerchfell 200, voneinander getrennt und/oder separiert werden, bzw. EMG-Störkomponenten unterdrückt werden.

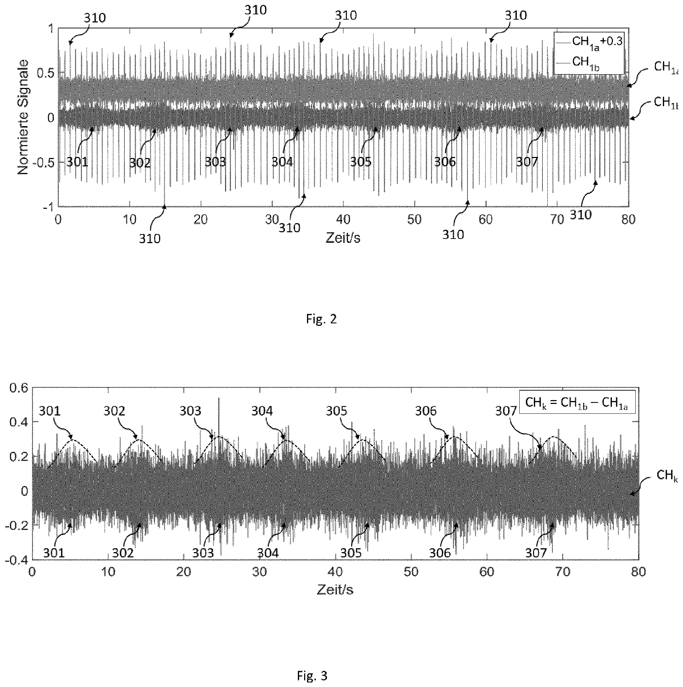

Fig. 2 zeigt ein beispielhaftes erstes Signal $CH_{1a} = EMG_{1a} + EKG$ und ein beispielhaftes zweites Signal $CH_{1b} = EMG_{1b} + EKG$, die beispielsweise mittels der Anordnung aus Fig. 1 erfasst werden können, wobei auf der X-Achse die Zeit in Sekunden und auf der Y-Achse die Signalstärke in einer beliebigen Einheit, z.B. optional eine normierte Signalstärke, z.B. eine Spannungsamplitude in mV, dargestellt sind. Zur Verdeutlichung bzw. zur besseren (visuellen) Unterscheidung dieser beiden Signale in einem Diagramm wurde das erste Signal $CH_{1a}$ mit einem Offset versehen, um z.B. die Signalen getrennt voneinander darstellen zu können. In Fig. 2 beträgt der Offset für das erste Signal $CH_{1a}$ 0,3, wodurch das erste Signal $CH_{1a}$ in dem Diagramm "nach oben" wandert. Ohne Offset wären diese beiden Signale jedoch überlagert, beispielsweise auf der gleichen Position.

Wie deutlich in Fig. 2 zu erkennen ist, weisen das erste Signal $CH_{1a}$ und das zweite Signal $CH_{1b}$ jeweils EMG-Signalanteile 301-307 (nur für das Signal $CH_{1b}$ markiert, jedoch auch im Signal $CH_{1a}$ vorhanden) und EMG-Störkomponenten 310 auf. Die EMG-Signalanteile 301-307 in Fig. 2 stellen eine Muskelaktivität z.B. des Zwerchfells 200 dar, z.B. eine oder mehrere Atemperioden eines Subjekts. In Fig. 2 sind insgesamt 7 Atemperioden 301-307 (s. auch Fig. 3) des Subjekts gemessen worden und/oder dargestellt. Die EMG-Signalkomponenten des ersten Signals $CH_{1a}$ (nicht markiert) und des zweiten Signals $CH_{1b}$ 310-307 sind jeweils im Vergleich zu den EMG-Störkomponenten 310, z.B. den EKG-Signalen bzw. den in Fig. 2 gezeigten R-Zacken 310 der EKG-Anteile 310 deutlich kleiner bzw. schwächer. Daher ist es schwierig und/oder aufwändig, die EMG-Signalanteile 301-307 akkurat zu erfassen und/oder diese von den EMG-Störanteilen 310 zu unterscheiden und/oder zu trennen.

Fig. 3 zeigt ein kombiniertes Signal $CH_k$ gemäß der Erfindung, dass auf Basis des ersten Signals $CH_{1a}$ und des zweiten Signals $CH_{1b}$, wie in Fig. 2 gezeigt, erhalten wird, wobei auf der X-Achse die Zeit in Sekunden und auf der Y-Achse die Signalstärke in einer beliebigen Einheit, z.B. optional eine normierte Signalstärke, z.B. eine Spannungsamplitude in mV, dargestellt sind. Insbesondere kann das kombinierte Signal $CH_k$ durch eine Subtraktion der ersten und zweiten Signale $CH_{1a}$ und $CH_{1b}$, z.B. gemäß Formel (2a) erhalten werden.

Wie bezüglich Fig. 2 erwähnt, ist bei dem kombinierten Signal $CH_k$ in Fig. 3 die myoelektrische Aktivität z.B. des Zwerchfells 200 deutlich zu erkennen, wobei alle EMG-Störkomponenten 310, z.B. die EKG-Signale 310 des Herzens 300, in dem kombinierten Signal $CH_k$, z.B. aufgrund der Subtraktion, z.B. gemäß Formel (2a) verschwunden sind. Dadurch lassen sich die 7 Atemperioden 301-307 des Subjekts deutlich erkennen, wobei diese Atemperioden 301-307, bzw. deren Peaks in myoelektrischer Aktivität, die von dem Atemvorgang resultierten, in dem kombinierten Signal $CH_k$ mittels gestrichelter Linien auf einer Seite, zur Verdeutlichung und aus Übersichtlichkeitsgründen, in dem Diagramm der Fig. 3 hervorgehoben sind.

Ferner sei anzumerken, dass sich durch kombinierte Messansätze, beispielweise durch Verwendungen einer oder mehrere duplizierter Sensoranordnungen 130, 140 (s. Fig. 1), wie hierin erwähnt, ein Signal-Rausch-Verhältnis weiter verbessern lässt. Es ermöglicht zudem eine räumliche und/oder zeitliche Repräsentation des Signalursprungs, wodurch die Zuordnung zum Zwerchfell und/oder anderen Muskelgruppen möglich ist.

Die in der vorstehenden Beschreibung, den Figuren und den Ansprüchen offenbarten Merkmale können sowohl einzeln als auch in beliebiger Kombination für die Realisierung der Erfindung in den verschiedenen Ausgestaltungen von Bedeutung sein.

**Patentansprüche**

1. Verfahren zur Separation von Nutz- und Störsignalen in einer elektromyografischen, EMG, -Messung in einer elektrisch aktiven Region von Muskeln und/oder zur Unterdrückung von Störsignalen in der EMG-Messung, das folgende Schritte umfasst:

   - Erfassen eines ersten Signals mit wenigstens einer ersten Sensoreinrichtung auf einer ersten Muskelgruppe, wobei das erste Signal eine erste EMG-Signalkomponente und eine erste EMG-Störkomponente aufweist;
   - Erfassen eines zweiten Signals mit wenigstens einer zweiten Sensoreinrichtung auf der ersten Muskelgruppe, wobei das zweite Signal eine zweite EMG-Signalkomponente und eine zweite EMG-Störkomponente aufweist;
   - Ermitteln eines kombinierten Signals auf Basis des ersten Signals und des zweiten Signals zur Unterdrückung der ersten und/oder der zweiten EMG-Störkomponente.

2. Verfahren nach Anspruch 1, wobei die ersten und zweiten Signale mittels der ersten Sensoreinrichtung und der zweiten Sensoreinrichtung jeweils örtlich aufgelöst, insbesondere unabhängig voneinander, insbesondere differentiell, erfasst werden.

3. Verfahren nach einem der Ansprüche 1-2, wobei das Ermitteln des kombinierten Signals eine Separation der ersten und/oder der zweiten EMG-Störkomponente umfasst, wobei die Separation auf einer Subtraktion des ersten und des zweiten Signals basiert.

4. Verfahren nach einem der Ansprüche 1-3, ferner umfassend:

   - optional, Visualisieren des kombinierten Signals, insbesondere eines Ursprungs des kombinierten Signals, wobei das Visualisieren insbesondere ein räumliches Visualisieren mittels einer Heatmap ist;
   - Anpassen von Signalamplituden, insbesondere Spannungsamplituden der ersten EMG-Störkomponente und Signalamplituden, insbesondere Spannungsamplituden, der zweiten EMG-Störkomponente,

   insbesondere derart, dass die Signalamplituden, insbesondere die Spannungsamplituden, der ersten EMG-Störkomponente im Wesentlichen den Signalamplituden, insbesondere den Spannungsamplituden, der zweiten EMG-Störkomponente entsprechen.

5. Verfahren nach Anspruch 4, wobei das Anpassen dynamisch und/oder kontinuierlich erfolgt, insbesondere während des Erfassens des ersten und des zweiten Signals und/oder zeitlich vor dem Ermitteln des kombinierten Signals.

6. Verfahren nach einem der Ansprüche 1-5, wobei

   - die erste EMG-Störkomponente und/oder die zweite EMG-Störkomponente ein Elektrokardiogramm, EKG, -Signal, insbesondere ein EKG-Spannungssignal, umfasst, wobei insbesondere die erste EMG-Störkomponente und/oder die zweite EMG-Störkomponente von einer gleichen Quelle verursacht werden und/oder ausgehen.

7. Verfahren nach einem der Ansprüche 1-6, ferner umfassend:

   - Anbringen der ersten Sensoreinrichtung und/oder der zweiten Sensoreinrichtung

-- auf unterschiedlichen Positionen der ersten Muskelgruppe, insbesondere benachbart, wobei die erste Muskelgruppe insbesondere Atemmuskeln umfasst, vorzugsweise ein Zwerchfell; und/oder
-- in einem im Wesentlichen identischen Abstand zu einer zweiten Muskelgruppe, wobei sich die zweite Muskelgruppe von der ersten Muskelgruppe unterscheidet, wobei die zweite Muskelgruppe insbesondere Herzmuskeln umfasst.

8. Verfahren nach Anspruch 7, wobei die erste Sensoreinrichtung und/oder die zweite Sensoreinrichtung eine Achse definieren, wobei die Achse im Wesentlichen in Richtung der zweiten Muskelgruppe zeigt.

9. Verfahren nach einem der Ansprüche 7-8, ferner umfassend:

Bereitstellen einer duplizierten Sensoranordnung, die eine dritte Sensoreinrichtung zum Erfassen eines dritten Signals und/oder der vierte Sensoreinrichtung zum Erfassen eines vierten Signals umfasst;
Anbringen der duplizierten Sensoranordnung auf der ersten Muskelgruppe und/oder wenigstens einer weiteren Muskelgruppe, die sich von der ersten Muskelgruppe unterscheidet.

10. Verfahren nach Anspruch 9, umfassend:

Ermitteln eines weiteren kombinierten Signals auf Basis des dritten Signals und des vierten Signals; und/oder
Visualisieren des kombinierten Signals und des weiteren kombinierten Signals, insbesondere mittels einer Heatmap, um eine räumliche und/oder zeitliche Auflösung zu erhalten.

11. Verfahren nach einem der Ansprüche 1-10, wobei

- sich die erste EMG-Signalkomponente von der zweiten EMG-Signalkomponente, insbesondere bezüglich eines, insbesondere zeitlichen, Signalverlaufs, insbesondere eines Spannungsverlaufs, unterscheidet; und/oder

die erste EMG-Signalkomponente und die zweite EMG-Signalkomponente einen im Wesentlichen identischen Informationsgehalt, insbesondere einen im Wesentlichen identischen Informationsgehalt bezüglich einer myoelektrischen Aktivität umfassen, oder eine im Wesentlichen Identische Verteilung der myoelektrischen Aktivität der ersten Muskelgruppe umfassen; und/oder
die erste Sensoreinrichtung von der zweiten Sensoreinrichtung auf der ersten Muskelgruppe beanstandet angeordnet ist, wobei der Abstand von einer Art und/oder eine Größe der ersten Muskelgruppe abhängt; und/oder
die erste Sensoreinrichtung und/oder die zweite Sensoreinrichtung jeweils wenigstens zwei Elektroden umfasst, zur Bereitstellung eines ersten differentiellen Messkanals und eines zweiten differentiellen Messkanals, wobei
die Elektroden Oberflächenelektroden und/oder Nadelelektroden sind.

12. Verwendung eines Verfahrens nach einem der Ansprüche 1-11 zum Monitoring von wenigstens einer Muskelgruppe, insbesondere Atemmuskeln, vorzugsweise zum Zwerchfell-Monitoring, und/oder zur Ansteuerung eines Beatmungsgerätes, und/oder zur Ansteuerung und/oder zum Monitoring von Prothesen und/oder Orthesen, und/oder zur Bewegungsanalyse.

13. Eine Vorrichtung zur Datenverarbeitung, umfassend Mittel zur Ausführung der Schritte des Verfahrens nach einem der Ansprüche 1-11.

14. Ein Computerprogrammprodukt, umfassend Befehle, die bei der Ausführung des Programms durch einen Computer diesen veranlassen, die Schritte des Verfahrens nach einem der Ansprüche 1-11 auszuführen.

15. Ein Computerlesbares Speichermedium, umfassend Befehle, die bei der Ausführung durch einen Computer diesen veranlassen, die Schritte des Verfahrens nach einem der Ansprüche 1-11 auszuführen.

Fig. 1

Fig. 2

Fig. 3

Europäisches
Patentamt

European
Patent Office

Office européen
des brevets

# EUROPÄISCHER RECHERCHENBERICHT

**Nummer der Anmeldung**

EP 24 19 5045

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2021 126194 A1 (FRAUNHOFER GES FORSCHUNG [DE]) 13. April 2023 (2023-04-13) * das ganze Dokument * * Absatz [0017] - Absatz [0095] * * Abbildungen 1-7 * * Ansprüche 1-13 * ----- | 1-15 | INV. A61B5/08 A61B5/00 A61B5/397 |
| X | DE 10 2019 203052 A1 (TECHNISCHE HOCHSCHULE LÜBECK [DE]) 10. September 2020 (2020-09-10) * Abbildung 1 * * Absatz [0005] - Absatz [0038] * ----- | 1-15 | |
| X | US 2003/188748 A1 (SINDERBY CHRISTER [CA] ET AL) 9. Oktober 2003 (2003-10-09) * Abbildungen 3-7 * * Absatz [0043] - Absatz [0053] * ----- | 1-15 | |
| X | CLANCY E A ET AL: "ESTIMATION AND APPLICATION OF EMG AMPLITUDE DURING DYNAMIC CONTRACTIONS", IEEE ENGINEERING IN MEDICINE AND BIOLOGY MAGAZINE, IEEE SERVICE CENTER, PISACATAWAY, NJ, US, Bd. 20, Nr. 6, 1. November 2001 (2001-11-01), Seiten 47-54, XP001177471, ISSN: 0739-5175, DOI: 10.1109/51.982275 * Seite 49, mittlere Spalte, Absatz 1 - Seite 51, rechte Spalte, Absatz 1 * ----- | 1-15 | RECHERCHIERTE SACHGEBIETE (IPC) A61B |

Der vorliegende Recherchenbericht wurde für alle Patentansprüche erstellt

| Recherchenort | Abschlußdatum der Recherche | Prüfer |
|---|---|---|
| Den Haag | 6. Januar 2025 | Knoop, Jan |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

...........................................................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 24 19 5045

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-01-2025

| Im Recherchenbericht angeführtes Patentdokument | Datum der Veröffentlichung | Mitglied(er) der Patentfamilie | Datum der Veröffentlichung |
|---|---|---|---|
| DE 102021126194 A1 | 13-04-2023 | DE 102021126194 A1 | 13-04-2023 |
| | | EP 4162878 A1 | 12-04-2023 |
| DE 102019203052 A1 | 10-09-2020 | DE 102019203052 A1 | 10-09-2020 |
| | | WO 2020178268 A1 | 10-09-2020 |
| US 2003188748 A1 | 09-10-2003 | AT E290338 T1 | 15-03-2005 |
| | | AT E521382 T1 | 15-09-2011 |
| | | AU 4126699 A | 20-12-1999 |
| | | CA 2239673 A1 | 04-12-1999 |
| | | CA 2336940 A1 | 09-12-1999 |
| | | DE 69924163 T2 | 29-12-2005 |
| | | EP 1082158 A1 | 14-03-2001 |
| | | EP 1366779 A1 | 03-12-2003 |
| | | EP 1366780 A1 | 03-12-2003 |
| | | EP 1382294 A2 | 21-01-2004 |
| | | US 7021310 B1 | 04-04-2006 |
| | | US 2003188748 A1 | 09-10-2003 |
| | | US 2003226565 A1 | 11-12-2003 |
| | | WO 9962580 A1 | 09-12-1999 |

EPO FORM P0461

Für nähere Einzelheiten zu diesem Anhang : siehe Amtsblatt des Europäischen Patentamts, Nr.12/82